# EUROPEAN PATENT SPECIFICATION

(11) **EP 4 317 969 B1**
(45) Date of publication and mention of the grant of the patent: **11.03.2026**
(21) Application number: 22775655.8
(22) Date of filing: 23.03.2022
(51) Int. Cl.: G01N 33/48

(54) **BLOOD SEPARATION COMPOSITION, BLOOD SAMPLING CONTAINER, AND METHOD FOR SEPARATING LEUKOCYTES**
BLUTTRENNUNGSZUSAMMENSETZUNG, BLUTPROBENBEHÄLTER UND VERFAHREN ZUR TRENNUNG VON LEUKOZYTEN
COMPOSITION DE SÉPARATION DE SANG, RÉCIPIENT D'ÉCHANTILLONNAGE DE SANG, ET PROCÉDÉ DE SÉPARATION DE LEUCOCYTES

(30) Priority: 24.03.2021 JP 2021049602
(43) Date of publication of application: 07.02.2024
(73) Proprietor: Sekisui Medical Co., Ltd., Tokyo 103-0027 (JP)
(72) Inventor: KOMAI, Kuniya, Tokyo 103-0027 (JP); INOUE, Tomonori, Tokyo 103-0027 (JP)
(74) Representative: Ter Meer Steinmeister & Partner
(86) International application number: PCT/JP2022/013383
(87) International publication number: WO 2022/202873

(56) References cited:
- WO-A1-2019/131613
- WO-A1-2021/010369
- JP-A- 2021 001 912
- US-A1- 2018 136 192
- EVONIK: "AEROSIL and SIPERNAT Silica: Versatile Raw Materials for Personal Care Formulations", 24 December 2012 (2012-12-24), pages 1 - 20, XP093246504, Retrieved from the Internet <URL:https://glenncorp.com/wp-content/uploads/2016/07/Aerosil_Sipernat.pdf> [retrieved on 20250204]

## Description

### TECHNICAL FIELD

The present invention relates to a blood separation composition. The present invention also relates to a blood sampling container in which the blood separation composition is accommodated in a blood sampling container body. The present invention further relates to a method for separating leukocytes using the blood sampling container.

### BACKGROUND ART

In clinical tests, a blood sampling container in which a blood separation composition is accommodated is widely used. By centrifuging the blood sampling container from which blood is sampled, a component contained in blood can be separated according to the specific gravity. For example, it is possible to separate blood into serum and blood clots or into plasma and a blood cell component. At this time, the blood separation composition is positioned between serum and blood clots or between plasma and a blood cell component, and functions as a partition wall for preventing mixing of these components.

As shown in Patent Documents 1 and 2 below, a blood separation composition capable of separating a specific blood cell component from blood is also known.

Patent Document 1 below describes a method for separating plasma, platelets, and leukocytes from blood using a gel-like substance (blood separation composition). In Patent Document 1, plasma, platelets, and leukocytes are separated above a partition wall formed of a gel-like substance. In Patent Document 1, as the gel-like substance, a gel-like substance containing silica powder is used.

Patent Document 2 below describes a method for separating a specific blood cell component from blood using a container accommodating a gel-like substance and a water-soluble density gradient material having specific gravity larger than that of the gel-like substance. Patent Document 2 describes that plasma, platelets, and leukocytes can be separated above a partition wall formed of a gel-like substance.

Patent Document 3 below describes another method for separating serum or plasma blood comprising a liquid organic compound and fine powder silica, wherein the fine powder silica includes hydrophobic silica having a hydrophobicity of 40%, 50% or 55% methanol wettability.

### Related Art Documents

### Patent Documents

Patent Document 1: US 4190535 A
Patent Document 2: JP S61-84557 A
Patent Document 3: US 2018/0136192 A1

### SUMMARY OF THE INVENTION

### PROBLEMS TO BE SOLVED BY THE INVENTION

Conventionally, as a blood separation composition, a blood separation composition containing fine powder silica has been widely used. After a blood sampling container is manufactured using the blood separation composition, the blood sampling container is stored for a certain period of time until it is used. In a blood sampling container in which a conventional blood separation composition containing fine powder silica is accommodated, the thixotropic index of the blood separation composition may gradually increase during storage. When the thixotropic index of the blood separation composition becomes excessively large, the formability of the partition wall may be deteriorated. When the partition wall by the blood separation composition is not satisfactorily formed, the blood component cannot be satisfactorily separated.

An object of the present invention is to provide a blood separation composition with which a partition wall can be satisfactorily formed even when the blood separation composition is stored for a long time. Another object of the present invention is to provide a blood sampling container accommodating the blood separation composition. Still another object of the present invention is to provide a method for separating leukocytes using the blood sampling container.

### MEANS FOR SOLVING THE PROBLEMS

According to a broad aspect of the present invention, there is provided a blood separation composition containing an organic component having fluidity at 25°C and fine powder silica, the fine powder silica including hydrophobic silica having a hydrophobicity of 60% or more as measured by a methanol wettability method comprising the following steps:
- adding and stirring 0.2 g of hydrophobic silica into 50 ml of aqueous methanol solutions of various concentrations;
- determining the solution with the lowest methanol concentration of the methanol solutions in which the entire amount of the hydrophobic silica has settled
- taking the methanol volume concentration of the determined solution as the hydrophobicity of the hydrophobic silica.

In a specific aspect of the blood separation composition according to the present invention, specific gravity at 25°C is 1.038 or more and 1.095 or less.

In a specific aspect of the blood separation composition according to the present invention, specific gravity at 25°C is 1.060 or more and 1.090 or less.

In a specific aspect of the blood separation composition according to the present invention, the organic component having fluidity at 25°C contains a resin.

In a specific aspect of the blood separation composition according to the present invention, the resin is a (meth)acrylic resin.

In a specific aspect of the blood separation composition according to the present invention, the fine powder silica includes hydrophilic silica.

In a specific aspect of the blood separation composition according to the present invention, the blood separation composition contains silicone oil.

According to a broad aspect of the present invention, there is provided a blood sampling container including: a blood sampling container body; and the above-described blood separation composition, the blood separation composition being accommodated in the blood sampling container body.

In a specific aspect of the blood sampling container according to the present invention, the blood sampling container includes a water-soluble density gradient material, in which the water-soluble density gradient material is accommodated in the blood sampling container body, and the water-soluble density gradient material is accommodated in the blood sampling container body on a bottom side of the blood sampling container body with respect to the blood separation composition.

In a specific aspect of the blood sampling container according to the present invention, the water-soluble density gradient material is Ficoll.

In a specific aspect of the blood sampling container according to the present invention, a density at 20°C of the water-soluble density gradient material is 1.083 g/mL or more and 1.085 g/mL or less.

According to a broad aspect of the present invention, there is provided a method for separating leukocytes using the above-described blood sampling container, the method comprising a centrifugation step of centrifuging the blood sampling container from which blood is sampled.

### EFFECT OF THE INVENTION

A blood separation composition according to the present invention contains an organic component having fluidity at 25°C and fine powder silica, the fine powder silica including hydrophobic silica having a hydrophobicity of 60% or more as measured by a methanol wettability method. Since the blood separation composition according to the present invention includes the above-described configuration, a partition wall can be satisfactorily formed even when the blood separation composition is stored for a long time.

### BRIEF DESCRIPTION OF DRAWINGS

[Fig. 1] Fig. 1 is a front cross-sectional view schematically illustrating a blood sampling container according to a first embodiment of the present invention.
[Fig. 2] Fig. 2 is a front cross-sectional view schematically illustrating a blood sampling container according to a second embodiment of the present invention.

### MODES FOR CARRYING OUT THE INVENTION

Hereinafter, the present invention will be described in detail.

A blood separation composition according to the present invention contains an organic component having fluidity at 25°C and fine powder silica, the fine powder silica including hydrophobic silica having a hydrophobicity of 60% or more as measured by a methanol wettability method.

Since the blood separation composition according to the present invention includes the above-described configuration, a partition wall can be satisfactorily formed even when the blood separation composition is stored for a long time.

After a blood sampling container is manufactured using the blood separation composition, the blood sampling container is stored for a certain period of time until it is used. In a blood sampling container in which a conventional blood separation composition containing fine powder silica is accommodated, the thixotropic index of the blood separation composition may gradually increase during storage. When the thixotropic index of the blood separation composition becomes excessively large, the formability of the partition wall may be deteriorated. For this reason, when blood is separated using the blood sampling container stored for a long time, the blood component cannot be satisfactorily separated due to the partition wall of the blood separation composition in some cases.

The present inventors have found that by using hydrophobic silica having a hydrophobicity of 60% or more, the formability of the partition wall can be improved even in the case of long-term storage. The reason why the above effect can be obtained by using the hydrophobic silica having a hydrophobicity of 60% or more is presumed to be that the aggregation of the fine powder silica is effectively suppressed, and as a result, an increase in thixotropic index of the blood separation composition during storage is suppressed, but is not limited thereto.

In the blood separation composition according to the present invention, by adjusting the specific gravity of the blood separation composition, a specific component can be separated from blood. In the blood separation composition according to the present invention, it is possible to separate blood into serum and blood clots or into plasma and a blood cell component. In the blood separation composition according to the present invention, leukocytes can be separated from blood. The blood separation composition according to the present invention may be used for separating serum or plasma from blood, or may be used for separating leukocytes from blood.

Hereinafter, details and the like of the components contained in the blood separation composition according to the present invention will be described. In the present specification, "(meth)acryl" means one or both of "acryl" and "methacryl".

### (Organic component having fluidity at 25°C)

The blood separation composition contains an organic component having fluidity at 25°C (hereinafter, sometimes abbreviated as "organic component"). Only one kind of the organic component may be used, or two or more kinds thereof may be used in combination.

In the present specification, the phrase "having fluidity at 25°C" means the viscosity at 25°C is 500 Pa·s or less.

The viscosity at 25°C of the organic component is preferably 30 Pa·s or more and more preferably 50 Pa·s or more, and is preferably 200 Pa·s or less and more preferably 100 Pa·s or less. When the viscosity is the lower limit or more and the upper limit or less, the fluidity of the blood separation composition is enhanced, and the strength of the partition wall can be increased.

The viscosity at 25°C of the organic component is measured using an E-type viscometer (for example, "TVE-35" manufactured by Toki Sangyo Co., Ltd.) under the conditions of 25°C and a shear rate of 1.0 second⁻¹.

Examples of the organic component include a resin, and a mixture of a resin and an organic compound such as a plasticizer. The organic component preferably contains a resin, and more preferably contains a resin and an organic compound. The organic component may be a resin (resin having fluidity at 25°C). When the organic component is a mixture of a resin and an organic compound, the mixture (organic component) may have fluidity, and the resin or the organic compound may not have fluidity. When the organic component is a mixture of a resin and an organic compound, the resin may be, for example, a resin that is a solid at 25°C. Only one kind of each of the resin and the organic compound may be used, or two or more kinds thereof may be used in combination.

From the viewpoint of more effectively exhibiting the effect of the present invention, the organic component preferably contains a resin, and more preferably is a mixture of a resin and an organic compound. When the organic component contains two or more components (including a case where the organic component contains a solid component and a liquid component), these components may be mixed separately in a step of producing a blood separation composition.

Examples of the resin include petroleum resin, a cyclopentadiene-based resin, a polyester resin, a polyurethane resin, a (meth)acrylic resin, a silicone resin, an α-olefin-fumaric acid ester copolymer, a copolymer of sebacic acid, 2,2-dimethyl-1,3-propanediol, and 1,2-propanediol, a polyether polyurethane-based resin, and a polyether polyester-based resin. Only one kind of the resin may be used, or two or more kinds thereof may be used in combination.

Examples of the petroleum resin include resins obtained by steam cracking of petroleums. Examples of the petroleum resin include homopolymers or copolymers of compounds (such as cyclopentadiene, isoprene, piperylene, and 2-methylbutene-1,2-methylbutene-2) contained in a Cs fraction; homopolymers or copolymers of compounds (such as styrene, vinyltoluene, α-methylstyrene, indene, cumarone) contained in a C₉ fraction; and copolymers of compounds contained in a Cs fraction and compounds contained in a C₉ fraction. The petroleum resin may be an unhydrogenated resin, a partially hydrogenated resin, or a completely hydrogenated resin.

Examples of commercially available products of the petroleum resin include "Regalite S5090" manufactured by Eastman Chemical Company.

Examples of the cyclopentadiene-based resin include a polymer of a cyclopentadiene-based monomer, a copolymer of a cyclopentadiene-based monomer and an aromatic monomer, and a dicyclopentadiene resin. The cyclopentadiene-based resin may or may not be hydrogenated. The polymer of a cyclopentadiene-based monomer and the copolymer of a cyclopentadiene-based monomer and an aromatic monomer may be oligomers.

Examples of the cyclopentadiene-based monomer include cyclopentadiene, dicyclopentadiene, and an alkyl-substituted derivative of cyclopentadiene.

Examples of the aromatic monomer include styrene, methylstyrene, indene, and methylindene.

Examples of commercially available products of the polymer of a cyclopentadiene-based monomer include "ESCOREZ 5380", "ESCOREZ 5300", "ESCOREZ 5320", "ESCOREZ 5340", "ESCOREZ 5400", and "ESCOREZ ECR251" manufactured by Exxon Mobil Corporation.

Examples of commercially available products of the copolymer of a cyclopentadiene-based monomer and an aromatic monomer include "ESCOREZ ECR227E", "ESCOREZ ECR235E", "ESCOREZ ECR231C", "ESCOREZ 5690", and "ESCOREZ 5600" manufactured by Exxon Mobil Corporation.

Examples of commercially available products of the dicyclopentadiene resin include SUKOREZ SU500" and "SUKOREZ SU90" manufactured by Kolon Industries. Inc.

Examples of the polyester resin include a polyalkylene terephthalate resin and a polyalkylene naphthalate resin. Examples of the polyalkylene terephthalate resin include polyethylene terephthalate, polybutylene terephthalate, and poly-1,4-cyclohexanedimethylene terephthalate.

Examples of the polyurethane resin include a reaction product of a polyol compound and an isocyanate compound.

Examples of the (meth)acrylic resin include a resin obtained by polymerizing at least one (meth)acrylic acid ester monomer, and a resin obtained by polymerizing at least one (meth)acrylic acid ester monomer and a monomer other than the at least one (meth)acrylic acid ester monomer. That is, examples of the (meth) acrylic resin include a polymer of a (meth) acrylic acid ester monomer and a polymer of a (meth)acrylic acid ester monomer and a monomer other than the (meth)acrylic acid ester monomer.

Examples of the (meth)acrylic acid ester monomer include (meth)acrylic acid alkyl ester, (meth)acrylic acid polyalkylene glycol ester, (meth)acrylic acid alkoxyalkyl ester, (meth)acrylic acid hydroxyalkyl ester, (meth)acrylic acid glycidyl ester, (meth)acrylic acid dialkylaminoalkyl ester, (meth)acrylic acid benzyl ester, (meth)acrylic acid phenoxyalkyl ester, (meth)acrylic acid cyclohexyl ester, (meth)acrylic acid isobornyl ester, and (meth)acrylic acid alkoxysilylalkyl ester. The number of carbon atoms of the alkyl group in the (meth)acrylic acid alkyl ester is preferably 1 or more and preferably 20 or less. Only one kind of the (meth)acrylic acid ester monomer may be used, or two or more kinds thereof may be used in combination.

Two or more kinds of the (meth)acrylic acid ester monomer are preferably used. When two or more kinds of the (meth)acrylic acid ester monomer are used, the content ratio of (meth)acrylic acid ester monomers having different molecular structures can be adjusted, so that the specific gravity and viscosity of a (meth) acrylic acid ester-based polymer to be obtained can be easily adjusted.

The content rate of the (meth)acrylic acid ester monomer in 100 wt% of the (meth)acrylic acid ester-based polymer is preferably 50 wt% or more, more preferably 60 wt% or more, even more preferably 70 wt% or more, further preferably 80 wt% or more, and particularly preferably 90 wt% or more. The upper limit of the content rate of the (meth)acrylic acid ester monomer in 100 wt% of the (meth)acrylic acid ester-based polymer is not particularly limited. The content rate of the (meth)acrylic acid ester monomer in 100 wt% of the (meth)acrylic acid ester-based polymer may be 100 wt% (total amount), less than 100 wt%, 90 wt% or less, or 80 wt% or less. When the content rate of the (meth)acrylic acid ester monomer is the lower limit or more, the viscosity of the blood separation composition can be further improved, and the strength of the partition wall can be further increased.

Examples of the monomer other than the (meth)acrylic acid ester monomer include a radically polymerizable monomer that can be radically copolymerized with the (meth)acrylic acid ester monomer.

Examples of the radically polymerizable monomer include an aromatic vinyl monomer, vinyl esters, vinyl ethers, vinyl pyrrolidone, and (meth)allyl ethers. Only one kind of the radically polymerizable monomer may be used, or two or more kinds thereof may be used in combination.

Examples of the aromatic vinyl monomer include styrene, α-methylstyrene, p-methylstyrene, α-methyl-p-methylstyrene, p-methoxystyrene, o-methoxystyrene, 2,4-dimethylstyrene, chlorostyrene, and bromostyrene.

Examples of the vinyl esters include (meth)acrylic acid, maleic anhydride, fumaric acid, (meth)acrylamide, (meth)acryldialkylamide, and vinyl acetate.

The weight average molecular weight (Mw) of the (meth)acrylic resin is preferably 19000 or more and more preferably 20000 or more, and is preferably 40000 or less and more preferably 30000 or less. When the weight average molecular weight is the lower limit or more, foaming of the blood separation composition during sterilization of the blood sampling container can be effectively suppressed. When the weight average molecular weight is the upper limit or less, the viscosity of the blood separation composition can be further improved, and the partition wall can be further satisfactorily formed.

The weight average molecular weight (Mw) refers to a weight average molecular weight in terms of polystyrene measured by gel permeation chromatography (GPC).

From the viewpoint of more effectively exhibiting the effect of the present invention, the resin preferably contains a petroleum resin, a cyclopentadiene-based resin, a polyester resin, or a (meth)acrylic resin, more preferably contains a (meth)acrylic resin, and further preferably is a (meth)acrylic resin.

Examples of the organic compound include a benzene polycarboxylic acid alkyl ester derivative. The organic compound is preferably a benzene polycarboxylic acid alkyl ester derivative. Therefore, the organic component is preferably a mixture of the resin and the benzene polycarboxylic acid alkyl ester derivative.

Examples of the benzene polycarboxylic acid alkyl ester derivative include phthalic acid ester, trimellitic acid ester, and pyromellitic acid ester. Only one kind of the benzene polycarboxylic acid alkyl ester derivative may be used, or two or more kinds thereof may be used in combination.

Examples of the trimellitic acid ester include tri-n-octyl trimellitate, triisooctyl trimellitate, and triisodecyl trimellitate.

Examples of the pyromellitic acid ester include tetraisooctyl pyromellitate.

Examples of commercially available products of the trimellitic acid ester include "MONOCIZER W700" and "MONOCIZER W-750" manufactured by DIC Corporation, and "SANSO CIZER TOTM" and "SANSO CIZER TITM" manufactured by New Japan Chemical Co., Ltd.

Examples of commercially available products of the pyromellitic acid ester include "MONOCIZER W-7010" manufactured by DIC Corporation.

The benzene polycarboxylic acid alkyl ester derivative is preferably phthalic acid ester, trimellitic acid ester, or pyromellitic acid ester, and is more preferably trimellitic acid ester.

Examples of the organic component also include a liquid mixture of a poly-α-pinene polymer and chlorinated hydrocarbon, a liquid mixture of chlorinated polybutene and epoxidized animal and vegetable oil, a liquid mixture of ethylene trifluoride chloride, a benzene polycarboxylic acid alkyl ester derivative, or the like and polyoxyalkylene glycol or the like, and a mixture composed of a liquid/liquid or a solid/liquid combination of a petroleum resin, a dicyclopentadiene resin, or the like and a benzene polycarboxylic acid alkyl ester derivative or the like.

The specific gravity at 25°C of the organic component is preferably 1.020 or more and more preferably 1.030 or more, and is preferably 1.080 or less, more preferably 1.060 or less, and further preferably 1.050 or less. When the specific gravity is the upper limit or less, the specific gravity of the blood separation composition is not too high, and the partition wall can be satisfactorily formed. When the specific gravity is the lower limit or more, the specific gravity of the blood separation composition can be satisfactorily adjusted without adding a large amount of the inorganic fine powder.

The specific gravity at 25°C of the organic component is measured by sequentially adding one drop of the organic component dropwise into saline at 25°C whose specific gravity is adjusted stepwise at intervals of 0.002, and by flotation and sedimentation in saline.

The content of the organic component in 100 wt% of the blood separation composition is preferably 80 wt% or more, more preferably 85 wt% or more, and further preferably 90 wt% or more, and is preferably 97 wt% or less.

### (Fine powder silica)

The blood separation composition contains fine powder silica. Examples of the fine powder silica include natural silica and synthetic silica. Examples of the synthetic silica include hydrophilic silica and hydrophobic silica. From the viewpoint of stable quality, the fine powder silica is preferably synthetic silica, and more preferably synthetic silica prepared by a gas phase method.

The fine powder silica contains hydrophobic silica having a hydrophobicity of 60% or more as measured by a methanol wettability method (hereinafter, sometimes referred to as "hydrophobic silica (X)"). Therefore, the fine powder silica includes the hydrophobic silica (X). The fine powder silica may include only the hydrophobic silica (X), or may include the hydrophobic silica (X) and fine powder silica other than the hydrophobic silica (X). The fine powder silica preferably includes the hydrophobic silica (X) and hydrophilic silica. The fine powder silica may include hydrophobic silica having a hydrophobicity of less than 60% as measured by a methanol wettability method as long as the effect of the present invention is not impaired. However, it is most preferable that the fine powder silica does not include hydrophobic silica having a hydrophobicity of less than 60% as measured by a methanol wettability method.

The average particle size of the fine powder silica is not particularly limited. The average particle size of the fine powder silica may be 1 nm or more or 10 nm or more, and may be 500 nm or less or 100 nm or less.

The average particle size of the fine powder silica and the hydrophobic silica (X) and hydrophilic silica, which are described below, is an average diameter as measured on a volume basis, and is a value of a median diameter (D50) of 50%. The volume average particle size (D50) can be measured by a laser diffraction/scattering method, an image analysis method, a Coulter method, a centrifugal sedimentation method, and the like. The volume average particle size (D50) is preferably determined by measurement by a laser diffraction/scattering method or an image analysis method.

The specific surface area of the fine powder silica is not particularly limited. The specific surface area of the fine powder silica may be 20 m²/g or more or 100 m²/g or more, and may be 500 m²/g or less or 300 m²/g or less.

The specific surface area of the fine powder silica and the hydrophobic silica (X) and hydrophilic silica, which are described below, is measured by a BET method.

### <Hydrophobic silica having hydrophobicity of 60% or more (hydrophobic silica (X))>

The blood separation composition contains the hydrophobic silica (X). Only one kind of the hydrophobic silica (X) may be used, or two or more kinds thereof may be used in combination.

From the viewpoint of exhibiting the effect of the present invention, the hydrophobicity of the hydrophobic silica (X) as measured by a methanol wettability method is 60% or more.

The hydrophobicity of the hydrophobic silica (X) as measured by a methanol wettability method is preferably 65% or more and more preferably 70% or more. When the hydrophobicity is the lower limit or more, the effect of the present invention can be more effectively exhibited. The hydrophobicity of the hydrophobic silica (X) as measured by a methanol wettability method is preferably as large as possible. The hydrophobicity of the hydrophobic silica (X) as measured by a methanol wettability method may be 95% or less or 90% or less.

The hydrophobicity of the hydrophobic silica as measured by a methanol wettability method is measured as follows. Methanol and water are mixed to prepare a methanol solution. The mixing ratio of methanol and water is changed to prepare a methanol solution in which the methanol concentration is adjusted at intervals of 5 vol%. The following operation is performed using the prepared methanol solutions having respective concentrations. In a 100 mL beaker, 50 mL of the methanol solution is put, and 0.2 g of hydrophobic silica is added. The methanol solution to which the hydrophobic silica is added is stirred using a magnetic stirrer. After the stirring is stopped, it is confirmed whether the entire amount of the hydrophobic silica settles to the bottom of the beaker. Among the concentrations (vol%) of the methanol solution when the entire amount of the hydrophobic silica has settled to the bottom of the beaker, the concentration (vol%) of the methanol solution having the lowest concentration is taken as the hydrophobicity (%) of the hydrophobic silica as measured by a methanol wettability method.

The average particle size of the hydrophobic silica (X) is not particularly limited. The average particle size of the hydrophobic silica (X) may be 1 nm or more or 10 nm or more, and may be 500 nm or less or 100 nm or less.

The specific surface area of the hydrophobic silica (X) is not particularly limited. The specific surface area of the hydrophobic silica (X) may be 20 m²/g or more or 100 m²/g or more, and may be 500 m²/g or less or 300 m²/g or less.

Examples of commercially available products of the hydrophobic silica (X) include RX200 and R812S (manufactured by Nippon Aerosil Co., Ltd.).

The content of the hydrophobic silica (X) in 100 wt% of the fine powder silica is preferably 20 wt% or more and more preferably 50 wt% or more, and is preferably 97 wt% or less and more preferably 95 wt% or less. When the content of the hydrophobic silica (X) is the lower limit or more and the upper limit or less, the effect of the present invention can be more effectively exhibited.

The content of the hydrophobic silica (X) in 100 wt% of the blood separation composition is preferably 1 wt% or more and more preferably 2 wt% or more, and is preferably 15 wt% or less and more preferably 10 wt% or less. When the content of the hydrophobic silica (X) is the lower limit or more and the upper limit or less, the effect of the present invention can be more effectively exhibited.

### <Hydrophilic silica>

The blood separation composition preferably contains hydrophilic silica. The fine powder silica preferably includes the hydrophilic silica. The hydrophilic silica usually has a hydroxyl group on the particle surface. The hydrophilic silica has an action of imparting thixotropy to the blood separation composition and adjusting the specific gravity by hydrogen bonding between hydroxyl groups on the particle surface. Therefore, by using the hydrophilic silica, it is easy to maintain both the specific gravity and the thixotropy of the blood separation composition in a suitable range.

The average particle size of the hydrophilic silica is not particularly limited. The average particle size of the hydrophilic silica may be 1 nm or more or 10 nm or more, and may be 500 nm or less or 100 nm or less.

The specific surface area of the hydrophilic silica is not particularly limited. The specific surface area of the hydrophilic silica may be 20 m²/g or more or 100 m²/g or more, and may be 500 m²/g or less or 300 m²/g or less.

Examples of commercially available products of the hydrophilic silica include AEROSIL series (manufactured by Nippon Aerosil Co., Ltd.) such as AEROSIL (registered trademark) 90G, 130, 200, 300, 200CF, and 300CF, REOLOSIL series (manufactured by Tokuyama Corporation) such as REOLOSIL (registered trademark) QS-10, QS-20, and QS-30, and WACKER HDK series (manufactured by Wacker Asahikasei Silicone Co., Ltd.) such as WACKER HDK S13, N20, and T30. The commercially available product of the hydrophilic silica is hydrophilic silica prepared by a gas phase method, and is easy to use.

The content of the hydrophilic silica in 100 wt% of the fine powder silica is preferably 1 wt% or more and more preferably 5 wt% or more, and is preferably 50 wt% or less and more preferably 20 wt% or less. When the content of the hydrophilic silica is the lower limit or more and the upper limit or less, both the specific gravity and the thixotropy of the blood separation composition can be maintained in a more suitable range.

The content of the hydrophilic silica in 100 wt% of the blood separation composition is preferably 0.2 wt% or more and more preferably 0.4 wt% or more, and is preferably 5 wt% or less and more preferably 2 wt% or less. When the content of the hydrophilic silica is the lower limit or more and the upper limit or less, both the specific gravity and the thixotropy of the blood separation composition can be maintained in a more suitable range.

The content of the fine powder silica in 100 wt% of the blood separation composition is preferably 1 wt% or more and more preferably 2 wt% or more, and is preferably 15 wt% or less and more preferably 10 wt% or less. When the content of the fine powder silica is the lower limit or more and the upper limit or less, both the specific gravity and the thixotropy of the blood separation composition can be maintained in a more suitable range.

### (Inorganic fine powder different from fine powder silica)

The blood separation composition may or may not contain an inorganic fine powder different from the fine powder silica (hereinafter, sometimes abbreviated as "inorganic fine powder"). The inorganic fine powder is used as a specific gravity adjusting component. When the specific gravity at 25°C of the blood separation composition using only fine powder silica is adjusted to 1.060 or more, the viscosity and the thixotropic index of the blood separation composition may become too high. In this case, the blood separation composition cannot exhibit sufficient fluidity at the time of centrifugation, and the partition wall may not be satisfactorily formed. Therefore, when a blood separation composition having specific gravity at 25°C of 1.060 or more is prepared, the blood separation composition preferably contains the inorganic fine powder. However, even when a blood separation composition having specific gravity at 25°C of 1.060 or more is prepared, the blood separation composition may not contain the inorganic fine powder. Even when a blood separation composition having specific gravity at 25°C of less than 1.060 is prepared, the blood separation composition may contain the inorganic fine powder. Only one kind of the inorganic fine powder may be used, or two or more kinds thereof may be used in combination.

Examples of the inorganic fine powder include titanium oxide powder, calcium carbonate powder, zinc oxide powder, alumina powder, glass fine powder, talc powder, kaolin powder, bentonite powder, titania powder, and zirconium powder.

From the viewpoint of maintaining the specific gravity and the thixotropy of the blood separation composition in a suitable range, the inorganic fine powder is preferably calcium carbonate powder, titanium oxide powder, or zinc oxide powder.

The specific gravity of the inorganic fine powder is preferably 3 or more, more preferably 3.5 or more, and further preferably 4 or more. The specific gravity of the inorganic fine powder is preferably as large as possible. When the specific gravity is the lower limit or more, the specific gravity of the blood separation composition can be effectively increased.

The average particle size of the inorganic fine powder is not particularly limited. The average particle size of the inorganic fine powder may be 10 nm or more or 100 nm or more, and may be 10 µm or less or 1 um or less.

The average particle size of the inorganic fine powder is an average diameter as measured on a volume basis, and is a value of a median diameter (D50) of 50%. The volume average particle size (D50) can be measured by a laser diffraction/scattering method, an image analysis method, a Coulter method, a centrifugal sedimentation method, and the like. The volume average particle size (D50) of the inorganic fine powder is preferably determined by measurement by a laser diffraction/scattering method or an image analysis method.

When the blood separation composition contains the inorganic fine powder, the content of the inorganic fine powder in 100 wt% of the blood separation composition is preferably 0.1 wt% or more and more preferably 0.3 wt% or more, and is preferably 10 wt% or less and more preferably 7 wt% or less. When the content of the inorganic fine powder is the lower limit or more and the upper limit or less, the effect of the present invention can be more effectively exhibited. When the content of the inorganic fine powder is the lower limit or more and the upper limit or less, the specific gravity of the blood separation composition can be effectively increased.

### (Silicone oil)

The blood separation composition preferably contains silicone oil. When the blood separation composition contains silicone oil, the thixotropy and the dispersibility of the fine powder silica can be improved, and the occurrence of phase separation can be suppressed. Only one kind of the silicone oil may be used, or two or more kinds thereof may be used in combination.

Examples of the silicone oil include dimethyl silicone oil, methyl phenyl silicone oil, methyl hydrogen silicone oil, alkyl-modified silicone oil, aralkyl-modified silicone oil, fluorine-modified silicone oil, polyether-modified silicone oil, amino-modified silicone oil, epoxy-modified silicone oil, phenol-modified silicone oil, carboxy-modified silicone oil, methacrylate-modified silicone oil, and alkoxy-modified silicone oil.

Examples of commercially available products of the dimethyl silicone oil include BY16-873 and PRX413 (manufactured by Dow Corning Toray Co., Ltd.).

Examples of commercially available products of the methyl phenyl silicone oil include SH510-100CS, SH510-500CS, SH550, and SH710 (manufactured by Dow Corning Toray Co., Ltd.).

Examples of commercially available products of the methyl hydrogen silicone oil include SH1107 (manufactured by Dow Corning Toray Co., Ltd.).

Examples of commercially available products of the alkyl-modified silicone oil include SH203, SH230, SF8416, and BY16-846 (manufactured by Dow Corning Toray Co., Ltd.).

Examples of commercially available products of the fluorine-modified silicone oil include FS1265-300CS, FS1265-1,000CS, and FS1265-10,000CS (manufactured by Dow Corning Toray Co., Ltd.).

Examples of the polyether-modified silicone oil include polyether-modified polyalkylsiloxane. Examples of commercially available products of the polyether-modified silicone oil include BY16-201, SF8410, SF8427, SF8428, FZ-2162, SH3746, SH3749, FZ-77, L-7001, Y7006, FZ-2104, FZ-2110, SH8400, SH8410, SH3773M, FZ-2207, FZ-2203, FZ-2222, and FZ-2208 (manufactured by Dow Corning Toray Co., Ltd.).

Examples of commercially available products of the amino-modified silicone oil include BY16-871, BY16-853U, FZ-3705, SF8417, BY16-849, FZ-3785, BY16-890, BY16-208, BY16-893, FZ-3789, BY16-878, and BY16-891 (manufactured by Dow Corning Toray Co., Ltd.).

Examples of commercially available products of the epoxy-modified silicone oil include BY16-855, SF8411, SF8413, BY16-839, and SF8421 (manufactured by Dow Corning Toray Co., Ltd.).

Examples of commercially available products of the carboxy-modified silicone oil include BY16-880 (manufactured by Dow Corning Toray Co., Ltd.).

The silicone oil is preferably alkyl-modified silicone oil, aralkyl-modified silicone oil, fluoro-modified silicone oil, or polyether-modified silicone oil, and is more preferably polyether-modified polyalkylsiloxane. In this case, the thixotropy and the dispersibility of the fine powder silica can be further improved, and the occurrence of phase separation can be further suppressed.

An HLB (Hydrophilic Lipophilic Balance) value of the silicone oil is preferably 1 or more, more preferably 2 or more, further preferably 4 or more, particularly preferably 5.5 or more, and most preferably 6 or more, and is preferably 10 or less, more preferably 8 or less, further preferably 7.5 or less, and particularly preferably 7 or less. When the HLB value is the lower limit or more, the thixotropy can be improved, and the blood separation composition hardly flows during storage of the blood sampling container. When the HLB value is the upper limit or less, the occurrence of phase separation can be further suppressed.

The HLB value represents an HLB value according to the Davies method. The HLB value is a value of 0 or more and 20 or less, and the smaller the HLB value, the stronger the hydrophobicity (lipophilicity), and the larger the HLB value, the stronger the hydrophilicity. The HLB value by the Davies method is calculated by the following equation.

### <HLB value = 7 + sum of the number of hydrophilic groups - sum of the number of lipophilic groups>

The number of groups is a unique numerical value defined for each functional group.

The content of the silicone oil in 100 wt% of the blood separation composition is preferably 0.01 wt% or more and more preferably 0.05 wt% or more, and is preferably 2.00 wt% or less and more preferably 0.50 wt% or less. When the content of the silicone oil is the lower limit or more and the upper limit or less, the occurrence of phase separation can be further suppressed.

### (Other components)

The blood separation composition may contain components other than the above-described components as long as the effect of the present invention is not impaired. Examples of the other components include an organic gelling agent, a thermoplastic elastomer, polyalkylene glycol, an auxiliary solvent, an antioxidant, a colorant, and water. Only one kind of each of the other components may be used, or two or more kinds thereof may be used in combination.

Examples of the organic gelling agent include dibenzylidene sorbitol, a dibenzylidene sorbitol derivative, and fatty acid amides. By containing the organic gelling agent, thixotropy can be further improved.

Examples of commercially available products of the dibenzylidene sorbitol and the dibenzylidene sorbitol derivative include "GEL ALL MD" and "GEL ALL D" manufactured by New Japan Chemical Co., Ltd.

Examples of the thermoplastic elastomer include a styrene-butadiene-styrene copolymer (SBS), a styrene-isoprene-styrene copolymer (SIS), a styrene-ethylene-butylene-styrene copolymer (SEBS), a styrene-butadiene-butylene-styrene copolymer (SBBS), a styrene-ethylene-propylene-styrene copolymer (SEPS), a styrene-butadiene copolymer (SB), a styrene-isoprene copolymer (SI), a styrene-ethylene-butylene copolymer (SEB), a styrene-butadiene-butylene copolymer (SBB), a styrene-ethylene-propylene copolymer (SEP), and modified products thereof.

Examples of the polyalkylene glycol include polybutylene glycol, polypropylene glycol, polyoxypropylene glyceryl ether, polyoxypropylene sorbitol, polyserine, polyoxypropylene diglyceryl ether, polyoxyethylene polyoxypropylene glycol, polyoxyethylene polyoxypropylene glyceryl ether, polyoxypropylene butyl ether, polyoxypropylene glycol monoether, polyoxypropylene alkyl ether, and modified products thereof.

Examples of the auxiliary solvent include toluene, N,N-dimethylformamide, 1-methyl-2-pyrrolidone, and dimethyl sulfoxide. By using the auxiliary solvent, for example, an organic gelling agent, a thermoplastic elastomer, and the like can be easily blended.

The blood separation composition may or may not contain water. The water is preferably purified water. The content of the water is preferably as small as possible.

### (Other details of blood separation composition)

By adjusting the specific gravity of the blood separation composition, a specific component can be separated from blood. For example, by using the blood separation composition, it is possible to separate blood into serum and blood clots or into plasma and a blood cell component. For example, by using the blood separation composition, it is possible to separate leukocytes (or plasma including leukocytes) from blood.

The specific gravity at 25°C of the blood separation composition may be 1.038 or more, 1.040 or more, 1.060 or more, 1.065 or more, 1.066 or more, or 1.070 or more. The specific gravity at 25°C of the blood separation composition may be 1.095 or less, 1.090 or less, 1.085 or less, 1.080 or less, 1.060 or less, 1.055 or less, 1.050 or less, or 1.045 or less.

From the viewpoint of separating blood into serum and blood clots, the specific gravity at 25°C of the blood separation composition is preferably 1.038 or more and more preferably 1.040 or more, and is preferably 1.095 or less, more preferably 1.085 or less, even more preferably 1.060 or less, further preferably 1.055 or less, and particularly preferably **1.050** or less. When the specific gravity is the lower limit or more and the upper limit or less, the partition wall having a favorable strength can be formed even at a low temperature or even when the centrifugal force is low. When the specific gravity is the lower limit or more and the upper limit or less, it is possible to satisfactorily separate serum with a small mixing amount of a blood cell component from blood.

From the viewpoint of separating blood into plasma and a blood cell component, the specific gravity at 25°C of the blood separation composition is preferably 1.038 or more and more preferably 1.040 or more, and is preferably 1.060 or less, more preferably 1.055 or less, and particularly preferably 1.050 or less. When the specific gravity is the lower limit or more and the upper limit or less, the partition wall having a favorable strength can be formed even at a low temperature or even when the centrifugal force is low. When the specific gravity is the lower limit or more and the upper limit or less, it is possible to satisfactorily separate plasma with a small mixing amount of a blood cell component from blood.

From the viewpoint of separating leukocytes (or plasma including leukocytes) from blood, the specific gravity at 25°C of the blood separation composition is preferably 1.060 or more, more preferably 1.065 or more, further preferably 1.066 or more, and particularly preferably 1.070 or more, and is preferably 1.090 or less, preferably 1.085 or less, and more preferably 1.080 or less. When the specific gravity is the lower limit or more and the upper limit or less, it is possible to satisfactorily obtain plasma with a small mixing amount of erythrocytes and a large content of leukocytes. When the specific gravity is the lower limit or more and the upper limit or less, the partition wall having a favorable strength can be formed even at a low temperature or even when the centrifugal force is low.

The specific gravity at 25°C of the blood separation composition is measured by sequentially adding one drop of the blood separation composition dropwise into saline at 25°C whose specific gravity is adjusted stepwise at intervals of 0.002, and by flotation and sedimentation in saline.

The viscosity at 25°C of the blood separation composition is preferably 100 Pa·s or more and more preferably 150 Pa·s or more, and is preferably 500 Pa·s or less, more preferably 400 Pa·s or less, and further preferably 350 Pa·s or less. When the viscosity is the lower limit or more and the upper limit or less, the effect of the present invention can be more effectively exhibited.

The viscosity at 25°C of the blood separation composition is measured using an E-type viscometer (for example, "TVE-35" manufactured by Toki Sangyo Co., Ltd.) under the conditions of 25°C and 0.5 rpm.

The thixotropic index at 25°C of the blood separation composition is preferably 1.10 or more and more preferably 1.15 or more, and is preferably 1.50 or less, more preferably 1.30 or less, further preferably 1.25 or less, and particularly preferably 1.20 or less. When the thixotropic index is the lower limit or more and the upper limit or less, the effect of the present invention can be more effectively exhibited. When the thixotropic index is the lower limit or more and the upper limit or less, even in a case where the blood separation composition is accommodated, for example, in the bottom of the blood sampling body, the blood separation composition exhibits favorable fluidity during centrifugation, and the partition wall can be more satisfactorily formed.

The thixotropic index at 25°C of the blood separation composition is determined as follows. The viscosity value of the blood separation composition measured using an E-type viscometer under the conditions of 25°C and 0.5 rpm is taken as a first viscosity value. The viscosity value of the blood separation composition measured using an E-type viscometer under the conditions of 25°C and 1.0 rpm is taken as a second viscosity value. A ratio of the first viscosity value to the second viscosity value (the first viscosity value/the second viscosity value) is taken as a thixotropic index at 25°C of the blood separation composition.

A method for producing the blood separation composition is not particularly limited. The blood separation composition can be produced, for example, by mixing the organic component having fluidity at 25°C, the fine powder silica, and other components blended as necessary. The mixing method is not particularly limited. The above components may be mixed by a known kneader such as a planetary mixer, a ball mill, or a disper.

### (Blood sampling container)

A blood sampling container according to the present invention includes a blood sampling container body and the above-described blood separation composition. In the blood sampling container according to the present invention, the blood separation composition is accommodated in the blood sampling container body.

The blood sampling container body preferably has an open end at one end and a closed bottom at the other end.

The material for the blood sampling container body is not particularly limited, and examples thereof include thermoplastic resins such as polyethylene (PE), polypropylene (PP), polystyrene (PS), polyethylene terephthalate (PET), polymethyl methacrylate, polyacrylonitrile, polyamide, an acrylonitrile-styrene copolymer, and an ethylene-vinyl alcohol copolymer; thermosetting resins such as an unsaturated polyester resin, an epoxy resin, and an epoxyacrylate resin; modified natural resins such as cellulose acetate, cellulose propionate, ethyl cellulose, and ethylchitin; silicates such as soda-lime glass, phosphosilicate glass, and borosilicate glass, glasses such as quartz glass, combinations thereof, and known materials mainly composed thereof.

The blood sampling container preferably includes a water-soluble density gradient material. In the blood sampling container, the water-soluble density gradient material is preferably accommodated in the blood sampling container body. When the blood sampling container is used for separating leukocytes from blood, the blood sampling container preferably includes the water-soluble density gradient material. In this case, for example, leukocytes can be floated in a water-soluble density gradient material layer after centrifugation. By using the water-soluble density gradient material, mixing of erythrocytes into the water-soluble density gradient material layer and the plasma can be effectively suppressed. Only one kind of the water-soluble density gradient material may be used, or two or more kinds thereof may be used in combination.

Examples of the water-soluble density gradient material include Ficoll and Percoll.

From the viewpoint of more effectively suppressing mixing of erythrocytes into the water-soluble density gradient material layer and the plasma, the water-soluble density gradient material is preferably Ficoll.

The density at 20°C of the water-soluble density gradient material is preferably 1.075 g/mL or more and more preferably 1.083 g/mL or more, and is preferably 1.095 g/mL or less and more preferably 1.085 g/mL or less. When the density is the lower limit or more and the upper limit or less, mixing of erythrocytes into the water-soluble density gradient material layer and the plasma can be more effectively suppressed.

The water-soluble density gradient material may be a solid at 25°C or a liquid at 25°C. The water-soluble density gradient material is preferably accommodated in a liquid state in the blood sampling container body. The water-soluble density gradient material may be accommodated in a state of being dissolved in the blood sampling container body.

The water-soluble density gradient material is preferably accommodated in the blood sampling container body on the bottom side of the blood sampling container body with respect to the blood separation composition. In the blood sampling container, it is preferable that the blood separation composition is positioned on the open end side of the blood sampling container body, and the water-soluble density gradient material is positioned on the bottom side of the blood sampling container body.

The blood sampling container may include known agents such as a blood clot adhesion preventing component and a blood coagulation accelerator according to the purpose of prevention of blood clot adhesion or acceleration of blood coagulation. For example, a blood clot adhesion preventing component or a blood coagulation accelerator may adhere to the inner wall of the blood sampling container body.

When the blood sampling container is used for separating plasma or leukocytes from blood, an anticoagulant is preferably accommodated in the blood sampling container body. The anticoagulant may be added to the sampled blood. Examples of the anticoagulant include heparin, ethylenediaminetetraacetic acid (EDTA), and citric acid.

The blood sampling container preferably includes a sealing member such as a plug and an aluminum seal. The sealing member is preferably provided at the open end of the blood sampling container body.

The internal pressure of the blood sampling container is not particularly limited. The blood sampling container can also be used as a vacuum blood sampling tube sealed by the sealing member after the inside is evacuated. In the case of the vacuum blood sampling tube, it is possible to easily sample a certain amount of blood regardless of the skills of the blood sampling operator.

From the viewpoint of preventing bacterial infection, the inside of the blood sampling container is preferably sterilized in compliance with the criteria of ISO or JIS. As a method for sterilizing the blood sampling container, a conventionally known method can be used. The blood sampling container is preferably sterilized by gamma ray sterilization, electron beam sterilization, high-pressure steam sterilization, or the like.

Hereinafter, specific embodiments of the present invention will be described with reference to the drawings. In the following drawings, the size, thickness, shape, and the like may be different from the actual size, thickness, shape, and the like for convenience of illustration.

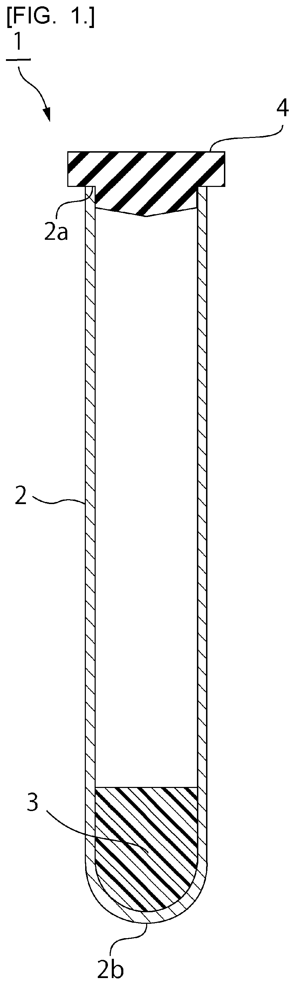

Fig. 1 is a front cross-sectional view schematically illustrating a blood sampling container according to a first embodiment of the present invention.

A blood sampling container 1 illustrated in Fig. 1 includes a blood sampling container body 2, a blood separation composition 3, and a plug 4. The blood sampling container body 2 has an open end 2a at one end and a bottom 2b at the other end. The blood separation composition 3 is accommodated in the bottom 2b of the blood sampling container body 2. The plug 4 is attached to the open end 2a of the blood sampling container body 2.

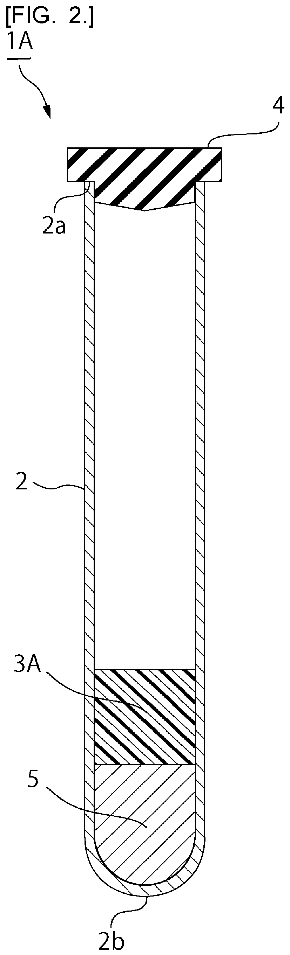

Fig. 2 is a front cross-sectional view schematically illustrating a blood sampling container according to a second embodiment of the present invention.

A blood sampling container 1A illustrated in Fig. 2 includes a blood sampling container body 2, a blood separation composition 3A, a water-soluble density gradient material 5, and a plug 4. Unlike the blood sampling container 1 illustrated in Fig. 1, the blood sampling container 1A illustrated in Fig. 2 includes the water-soluble density gradient material 5. The blood separation composition 3A is accommodated in the blood sampling container body 2 on the open end 2a side of the blood sampling container body 2 with respect to the water-soluble density gradient material 5. The water-soluble density gradient material 5 is accommodated in the blood sampling container body 2 on the bottom 2b side of the blood sampling container body 2 with respect to the blood separation composition 3A.

### (Method for separating blood component)

A specific blood component can be separated from blood using the blood sampling container.

The method for separating a blood component may be a method for separating serum from blood (method for separating serum), a method for separating plasma from blood (method for separating plasma), or a method for separating leukocytes from blood (method for separating leukocytes). By adjusting the specific gravity of the blood separation composition, a target blood component can be separated.

The method for separating a blood component (the method for separating serum, the method for separating plasma, or the method for separating leukocytes) preferably includes a centrifugation step of centrifuging the blood sampling container from which blood is sampled. The method for separating a blood component (the method for separating serum, the method for separating plasma, or the method for separating leukocytes) more preferably includes a blood sampling step of sampling blood in the blood sampling container body and a centrifugation step of centrifuging the blood sampling container from which blood is sampled.

In the method for separating plasma, in the blood sampling step, it is preferable that the anticoagulant is accommodated in the blood sampling container body or blood to which the anticoagulant is added is sampled in the blood sampling container body.

In the method for separating leukocytes, in the blood sampling step, it is preferable that the anticoagulant is accommodated in the blood sampling container body or blood to which the anticoagulant is added is sampled in the blood sampling container body.

In the method for separating serum, the blood clot is positioned below the partition wall formed by the blood separation composition, and the serum is positioned above the partition wall. In the method for separating plasma, the blood cell component is positioned below the partition wall formed by the blood separation composition, and the plasma is positioned above the partition wall.

In the method for separating leukocytes, the erythrocyte component is positioned below the partition wall formed by the blood separation composition, and the leukocyte is positioned above the partition wall. When the water-soluble density gradient material is not used, in the method for separating leukocytes, for example, leukocytes can be deposited on the partition wall formed by the blood separation composition. In the case of using the water-soluble density gradient material, in the method for separating leukocytes, for example, a water-soluble density gradient material layer containing leukocytes is positioned on the partition wall formed by the blood separation composition, and the plasma is positioned above the water-soluble density gradient material layer containing leukocytes

In the method for separating a blood component, centrifugation conditions in the centrifugation step are not particularly limited. Examples of the centrifugation conditions include a condition of performing centrifugal separation at 400 G or more and 4000 G or less for 10 minutes or more and 120 minutes or less.

Hereinafter, the present invention will be specifically described with reference to Examples and Comparative Examples. The present invention is not limited to the following Examples.

As materials for the blood separation composition, the following were prepared.

### (Organic component having fluidity at 25°C)

(Meth)acrylic resin (viscosity at 25°C: 75 Pa·s, specific gravity at 25°C: 1.033, weight average molecular weight: 20000, synthesized according to the following Synthesis Example 1)

### <Synthesis Example 1>

In the presence of an azo-based polymerization initiator, 2-ethylhexyl acrylate and butyl acrylate were radically polymerized by a solution polymerization method to obtain a (meth) acrylic resin having fluidity at 25°C. The viscosity at 25°C, the specific gravity at 25°C, and the weight average molecular weight of the obtained (meth)acrylic resin were measured by the methods described above.

### (Material for organic component having fluidity at 25°C)

### Resin:

Petroleum resin ("Regalite S5090" manufactured by Eastman Chemical Company)
Dicyclopentadiene resin 1 ("SUKOREZ SU500" manufactured by Kolon Industries. Inc.)
Dicyclopentadiene resin 2 ("SUKOREZ SU90" manufactured by Kolon Industries. Inc.)

### Organic compound:

Trimellitic acid ester (benzene polycarboxylic acid alkyl ester derivative, "MONOCIZER W700" manufactured by DIC Corporation)

### (Fine powder silica)

### Hydrophobic silica (X):

Hydrophobic silica 1 ("RX200" manufactured by Nippon Aerosil Co., Ltd., hydrophobicity as measured by a methanol wettability method: 70%, specific surface area: 140 m²/g)
Hydrophobic silica 2 ("R812S" manufactured by Nippon Aerosil Co., Ltd., hydrophobicity as measured by a methanol wettability method: 60%, specific surface area: 220 m²/g)

Hydrophobic silica not corresponding to hydrophobic silica (X) :
Hydrophobic silica 3 ("R805" manufactured by Nippon Aerosil Co., Ltd., hydrophobicity as measured by a methanol wettability method: 55%, specific surface area: 150 m²/g)
Hydrophobic silica 4 ("R812" manufactured by Nippon Aerosil Co., Ltd., hydrophobicity as measured by a methanol wettability method: 50%, specific surface area: 260 m²/g)
Hydrophobic silica 5 ("R974" manufactured by Nippon Aerosil Co., Ltd., hydrophobicity as measured by a methanol wettability method: 40%, specific surface area: 170 m²/g)
Hydrophilic silica ("200CF" manufactured by Nippon Aerosil Co., Ltd., specific surface area: 200 m²/g)

The hydrophobicity of each of the hydrophobic silicas 1 to 5 as measured by a methanol wettability method was measured by the method described above.

### (Inorganic fine powder other than fine powder silica)

Calcium carbonate powder ("SocalUP" manufactured by Imerys, specific gravity: 2.7, average particle size: 50 nm)
Titanium oxide powder ("A-100" manufactured by ISHIHARA SANGYO KAISHA, LTD., specific gravity: 4, average particle size: 150 nm)

### (Other components)

Organic gelling agent ("GEL ALL D" manufactured by New Japan Chemical Co., Ltd.)
1-Methyl -2 pyrrolidone (auxiliary solvent)
Silicone oil (polyether-modified silicone oil, "SF8410" manufactured by Dow Corning Toray Co., Ltd., HLB value: 6, polyether-modified polyalkylsiloxane)

### (Example 1)

### Preparation of blood separation composition:

The components shown in Table 1 were blended and mixed at blending ratios described in Table 1 to obtain a blood separation composition.

### Preparation of blood sampling container (1):

As the blood sampling container body, a polyethylene terephthalate tube (PET bottomed tube) having a length of 100 mm and an open end with an inner diameter of 14 mm was prepared. In the blood sampling container body, 1 g of the obtained blood separation composition was accommodated. The inside of the blood sampling container was depressurized to 30 kPa and sealed with a butyl rubber plug. In this way, a blood sampling container (1) in which the blood separation composition is accommodated in the blood sampling container body was produced.

### Preparation of blood sampling container (2):

As the blood sampling container body, a glass tube (glass bottomed tube) having a length of 125 mm and an open end with an inner diameter of 14 mm was prepared. As the water-soluble density gradient material, Ficoll ("Ficoll Paque premium 1.084" manufactured by Cytiva, density at 20°C: 1.084 g/mL) was prepared. In the blood sampling container body, 2 mL of Ficoll was accommodated, and then in the blood sampling container body, 3 g of the obtained blood separation composition was accommodated. As the anticoagulant, 1 mL of an aqueous sodium citrate solution was accommodated in the blood sampling container body. The inside of the blood sampling container was depressurized to 30 kPa and sealed with a butyl rubber plug. In this way, a blood sampling container (2) in which the Ficoll and the blood separation composition are accommodated in the blood sampling container body was produced. In the obtained blood sampling container (2), the blood separation composition is accommodated on the open end side of the blood sampling container body with respect to Ficoll, and the Ficoll is accommodated on the bottom side of the blood sampling container body with respect to the blood separation composition.

### Storage test (45°C and 75as RH for 4 weeks):

The obtained blood sampling containers (1) and (2) were stored in a thermo-hygrostat set at 45°C and 75% RH for 4 weeks. The above temperature and humidity conditions are accelerated test conditions for long-term storage stability.

### (Examples 2 to 4 and Comparative Examples 1 to 3)

A blood separation composition, a blood sampling container (1), and a blood sampling container (2) were produced in the same manner as in Example 1, except that the type and blending amount of the blending components were changed as shown in Tables 1 and 2. A storage test (45°C and 75% RH for 4 weeks) was performed in the same manner as in Example 1.

### (Example 5)

### Preparation of blood separation composition:

Materials of an organic component having fluidity at 25°C described in Table 1 were blended, heated and dissolved at 130°C, and mixed to prepare an organic component having fluidity at 25°C. Next, an organic component having fluidity at 25°C, fine powder silica, inorganic fine powder, and other components were mixed at a blending ratio described in Table 1 to prepare a blood separation composition.

### Preparation of blood sampling containers (1) and (2):

A blood sampling container (1) and a blood sampling container (2) were produced in the same manner as in Example 1, except that the obtained blood separation composition was used. A storage test (45°C and 75% RH for 4 weeks) was performed in the same manner as in Example 1.

### (Example 6 and Comparative Example 4)

A blood separation composition and a blood sampling container (1) were produced in the same manner as in Example 1, except that the type and blending amount of the blending components were changed as shown in Tables 1 and 2. A storage test (45°C and 75% RH for 4 weeks) was performed in the same manner as in Example 1. In Example 6 and Comparative Example 4, a blood sampling container (2) was not produced.

### (Evaluation)

### (1) Specific gravity at 25°C of blood separation composition

The specific gravity was measured by sequentially adding one drop of the obtained blood separation composition dropwise into saline at 25°C whose specific gravity was adjusted stepwise at intervals of 0.002, and by flotation and sedimentation in saline.

### (2) Viscosity and thixotropic index of blood separation composition

The viscosity of the blood separation composition immediately after production (blood separation composition before the storage test) was measured under the conditions of 25°C and 0.5 rpm using an E-type viscometer ("TVE-35" manufactured by Toki Sangyo Co., Ltd.) and taken as the viscosity (first viscosity value) of the blood separation composition before storage. The viscosity of the blood separation composition immediately after production (blood separation composition before the storage test) was measured under the conditions of 25°C and 1.0 rpm using an E-type viscometer ("TVE-35" manufactured by Toki Sangyo Co., Ltd.) and taken as a second viscosity value. The ratio of the first viscosity value to the second viscosity value (the first viscosity value/the second viscosity value) was calculated and taken as a thixotropic index at 25°C of the blood separation composition before storage.

The blood separation composition was sampled from the blood sampling container (1) after the storage test (45°C and 75% RH for 4 weeks). Using the sampled blood separation composition, the viscosity was measured under the conditions of 25°C and 0.5 rpm in the same manner as described above and was taken as the viscosity (first viscosity value) of the blood separation composition after storage. Using the sampled blood separation composition, the viscosity was measured under the conditions of 25°C and 1.0 rpm in the same manner as described above and was taken as the viscosity (second viscosity value) of the blood separation composition after storage. The ratio of the first viscosity value to the second viscosity value (the first viscosity value/the second viscosity value) was calculated and taken as a thixotropic index at 25°C of the blood separation composition after storage.

### (3) Partition wall formability (A)

The following test was performed using each of the blood sampling container (1) before the storage test and the blood sampling container (1) after the storage test. In the blood sampling container (1), 4 mL of blood with EDTA added was sampled. The blood sampling container (1) was then centrifuged at 1500 G for 30 minutes. The following tests (1A) and (2A) were performed to determine the partition wall formability according to the following criteria. In Examples 1 to 5, plasma containing leukocytes was separated above the partition wall, and leukocytes were deposited on the partition wall. In Example 6, plasma was separated above the partition wall.

Test (1A): The blood sampling container (1) after centrifugation was visually observed. A case where the blood separation composition was positioned between the erythrocyte layer and the plasma layer was determined as good. On the other hand, a case where the entire amount of the blood separation composition was positioned closer to the closed end side of the blood sampling container (1) than the erythrocyte layer and the partition wall was not formed was determined as defective.

Test (2A): The thickness of the partition wall formed by the blood separation composition was measured by a caliper.

### <Determination criteria for partition wall formability (A)>

○○: Among 20 blood sampling containers (1), the number of blood sampling containers (1) having a good determination result of the test (1A) is 20, and the number of blood sampling containers (1) having a minimum thickness of the partition wall of 5 mm or more measured in the test (2A) is 10 or more and 20 or less.

○: Among 20 blood sampling containers (1), the number of blood sampling containers (1) having a good determination result of the test (1A) is 20, and the number of blood sampling containers (1) having a minimum thickness of the partition wall of 5 mm or more measured in the test (2A) is 0 or more and 9 or less.

×: Among 20 blood sampling containers (1), the number of blood sampling containers (1) having a good determination result of the test (1A) is 19 or less.

### (4) Partition wall formability (B)

The following test was performed using each of the blood sampling container (2) before the storage test and the blood sampling container (2) after the storage test. In the blood sampling container (2), 8 mL of blood was sampled. The blood sampling container (2) was then centrifuged at 1800 G for 30 minutes. The following tests (1B) and (2B) were performed to determine the partition wall formability according to the following criteria. In Examples 1 to 5, a Ficoll layer was formed above the partition wall, plasma was separated above the Ficoll layer, and leukocytes were floating in the Ficoll layer.

Test (1B): The blood sampling container (2) after centrifugation was visually observed. A case where the blood separation composition was positioned closer to the open end of the blood sampling container (2) than the erythrocyte layer was determined as good. On the other hand, a case where the entire amount of the blood separation composition was positioned closer to the closed end side of the blood sampling container (2) than the erythrocyte layer and the partition wall was not formed was determined as defective.

Test (2B): The thickness of the partition wall formed by the blood separation composition was measured by a caliper.

### <Determination criteria for partition wall formability (B)>

○○: Among 20 blood sampling containers (2), the number of blood sampling containers (2) having a good determination result of the test (1B) is 20, and the number of blood sampling containers (2) having a minimum thickness of the partition wall of 5 mm or more measured in the test (2B) is 10 or more and 20 or less.

○: Among 20 blood sampling containers (2), the number of blood sampling containers (2) having a good determination result of the test (1B) is 20, and the number of blood sampling containers (2) having a minimum thickness of the partition wall of 5 mm or more measured in the test (2B) is 0 or more and 9 or less.

×: Among 20 blood sampling containers (2), the number of blood sampling containers (2) having a good determination result of the test (1B) is 19 or less.

Configurations and results are shown in Tables 1 and 2 below. In Examples 1 to 5, the blood cell component positioned above the partition wall formed by the blood separation composition was suspended in plasma positioned above the blood cell component, plasma containing the blood cell component was collected, and the recovery rate of leukocytes was calculated. As a result, the recovery rate of leukocytes was higher in the case of using the blood sampling container (2) in which Ficoll was accommodated than in the case of using the blood sampling container (1) in which Ficoll was not accommodated.

**[Table 1]**

| | | | | Example 1 | Example 2 | Example 3 | Example 4 | Example 5 | Example 6 |
|---|---|---|---|---|---|---|---|---|---|
| Organic component having fluidity at 25°C | | (Meth)acrylic resin | wt% | 92.15 | 92.15 | 91.35 | 93.15 | - | 97.4 |
| | | Petroleum resin | wt% | - | - | - | - | 13.80 | - |
| | | Dicyclopentadiene resin 1 | wt% | - | - | - | - | 17.10 | - |
| | | Dicyclopentadiene resin 2 | wt% | - | - | - | - | 18.70 | - |
| | | Trimellitic acid ester | wt% | - | - | - | - | 42.40 | - |
| Fine powder silica | | Hydrophobic silica 1 (hydrophobicity: 70%) | wt% | 6.7 | - | 7.5 | - | 6.8 | 1.7 |
| | | Hydrophobic silica 2 (hydrophobicity: 60%) | wt% | - | 6.7 | - | 6 | - | - |
| | | Hydrophobic silica 3 (hydrophobicity: 55%) | wt% | - | - | - | - | - | - |
| | | Hydrophobic silica 4 (hydrophobicity: 50%) | wt% | - | - | - | - | - | - |
| | | Hydrophobic silica 5 (hydrophobicity: 40%) | wt% | - | - | - | - | - | - |
| | | Hydrophilic silica | wt% | 0.7 | 0.7 | 0.5 | 0.7 | 0.5 | 0.5 |
| Inorganic fine powder different from fine powder silica | | Calcium carbonate powder | wt% | 0.3 | 0.3 | 0.5 | - | - | 0.1 |
| | | Titanium oxide powder | wt% | - | - | - | - | 0.3 | - |
| Other components | | Organic gelling agent | wt% | - | - | - | - | 0.1 | - |
| | | 1 MeLhyl 2 pyrrolidone | wt% | | | | | 0.3 | |
| | | Silicone oil | wt% | 0.15 | 0.15 | 0.15 | 0.15 | - | 0.30 |
| Total | | | wt% | 100 | 100 | 100 | 100 | 100 | 100 |
| Blood separation composition | Specific gravity at 25°C | | - | 1.078 | 1.078 | 1.083 | 1.070 | 1.076 | 1.047 |
| | Before storage | Viscosity (25°C and 0.5 rpm) | Pa·s | 288.6 | 304.1 | 306. 8 | 295.7 | 276. 9 | 253.7 |
| | | Thixotropic index (25°C) | - | 1.17 | 1.20 | 1.15 | 1.19 | 1.15 | 1.20 |
| | After storage (45°C and 75% RH, for 4 weeks) | Viscosity (25°C and 0.5 rpm) | Pa·s | 307.9 | 310.9 | 334.3 | 311.2 | 292.3 | 260.8 |
| | | Thixotropic index (25°C) | - | 1. 18 | 1.19 | 1. 18 | 1.19 | 1. 18 | 1.23 |
| Partition wall formability (A) (blood sampling container (1)) | | Before storage | - | ○○ | ○○ | ○○ | ○○ | ○○ | ○○ |
| | | After storage (45°C and 75% RH, for 4 weeks) | - | ○○ | ○ | ○○ | ○ | ○○ | ○○ |
| Partition wall formability (B) (blood sampling container (2)) | | Before storage | - | ○○ | ○○ | ○○ | ○○ | ○○ | - |
| | | After storage (45°C and 75% RH, for 4 weeks) | - | ○○ | ○ | ○○ | ○ | ○○ | - |

**[Table 2]**

| | | | | Comparative Example 1 | Comparative Example 2 | Comparative Example 3 | Comparative Example 4 |
|---|---|---|---|---|---|---|---|
| Organic component having fluidity at 25°C | | (Meth)acrylic resin | wt% | 92.15 | 92.15 | 93.45 | 97.4 |
| | | Petroleum resin | wt% | - | - | - | - |
| | | Dicyclopentadiene resin 1 | wt% | - | - | - | - |
| | | Dicyclopentadiene resin 2 | wt% | - | - | - | - |
| | | Trimellitic acid ester | wt% | - | - | - | - |
| Fine powder silica | | Hydrophobic silica 1 (hydrophobicity: 70%) | wt% | - | - | - | - |
| | | Hydrophobic silica 2 (hydrophobicity: 60%) | wt% | - | - | - | - |
| | | Hydrophobic silica 3 (hydrophobicity: 55%) | wt% | 6.7 | - | - | - |
| | | Hydrophobic silica 4 (hydrophobicity: 50%) | wt% | - | 6.7 | - | - |
| | | Hydrophobic silica 5 (hydrophobicity: 40%) | wt% | - | - | 2.45 | 1.7 |
| | | Hydrophilic silica | wt% | 0.7 | 0.7 | 0.75 | 0.5 |
| Inorganic fine powder different from fine powder silica | | Calcium carbonate powder | wt% | 0.3 | 0.3 | - | 0.1 |
| | | Titanium oxide powder | wt% | - | - | 3.2 | - |
| Other components | | Organic gelling agent | wt% | - | - | - | - |
| | | 1-Methyl -2 pyrrolidone | wt% | - | - | - | - |
| | | Silicone oil | wt% | 0.15 | 0.15 | 0.15 | 0.30 |
| Total | | | wt% | 100 | 100 | 100 | 100 |
| Blood separation composition | Specific gravity at 25°C | | - | 1.078 | 1.078 | 1.078 | 1.047 |
| | Before storage | Viscosity (25°C and 0.5 rpm) | Pa·s | 366.9 | 362.3 | 208.6 | 260.5 |
| | | Thixotropic index (25°C) | - | 1.21 | 1.19 | 1.20 | 1.23 |
| | After storage (45°C and 75% RH, for 4 weeks) | Viscosity (25°C and 0.5 rpm) | Pa·s | 390.6 | 385 | 236.2 | 302.0 |
| | | Thixotropic index (25°C) | - | 1.28 | 1.27 | 1.27 | 1.30 |
| Partition wall formability (A) (blood sampling container (1)) | | Before storage | - | ○○ | ○○ | ○○ | ○○ |
| | | After storage (45°C and 75% RH, for 4 weeks) | - | × | × | × | × |
| Partition wall formability (B) (blood sampling container (2)) | | Before storage | - | ○○ | ○○ | ○○ | - |
| | | After storage (45°C and 75% RH, for 4 weeks) | - | × | × | × | - |

### EXPLANATION OF SYMBOLS

1, 1A: Blood sampling container
2: Blood sampling container body
2a: Open end
2b: Bottom
3, 3A: Blood separation composition
4: Plug
5: Water-soluble density gradient material

## Claims

1. A blood separation composition comprising:
an organic component having fluidity at 25°C and fine powder silica,
**characterised in that** the fine powder silica includes hydrophobic silica having a hydrophobicity of 60% or more as measured by a methanol wettability method comprising the following steps:
- adding and stirring 0.2 g of hydrophobic silica into 50 ml of aqueous methanol solutions of various concentrations;
- determining the solution with the lowest methanol concentration of the methanol solutions in which the entire amount of the hydrophobic silica has settled
- taking the methanol volume concentration of the determined solution as the hydrophobicity of the hydrophobic silica.

2. The blood separation composition according to claim 1, wherein specific gravity at 25°C is 1.038 or more and 1.095 or less.

3. The blood separation composition according to claim 1 or 2, wherein specific gravity at 25°C is 1.060 or more and 1.090 or less.

4. The blood separation composition according to any one of claims 1 to 3, wherein the organic component having fluidity at 25°C contains a resin.

5. The blood separation composition according to claim 4, wherein the resin is a (meth)acrylic resin.

6. The blood separation composition according to any one of claims 1 to 5, wherein the fine powder silica includes hydrophilic silica.

7. The blood separation composition according to any one of claims 1 to 6, comprising silicone oil.

8. A blood sampling container comprising:
a blood sampling container body; and
the blood separation composition according to any one of claims 1 to 7,
the blood separation composition being accommodated in the blood sampling container body.

9. The blood sampling container according to claim 8, comprising a water-soluble density gradient material,
wherein the water-soluble density gradient material is accommodated in the blood sampling container body, and
the water-soluble density gradient material is accommodated in the blood sampling container body on a bottom side of the blood sampling container body with respect to the blood separation composition.

10. The blood sampling container according to claim 9, wherein the water-soluble density gradient material is Ficoll.

11. The blood sampling container according to claim 9 or 10, wherein a density at 20°C of the water-soluble density gradient material is 1.083 g/mL or more and 1.085 g/mL or less.

12. A method for separating leukocytes using the blood sampling container according to any one of claims 8 to 11, the method comprising a centrifugation step of centrifuging the blood sampling container from which blood is sampled.

## Patentansprüche

1. Blutseparationszusammensetzung, umfassend:
eine organische Komponente, die bei 25°C fließfähig ist, und feines Siliciumdioxidpulver, **dadurch gekennzeichnet, dass** das feine Siliciumdioxidpulver hydrophobes Siliciumdioxid mit einer Hydrophobie von 60% oder mehr enthält, gemessen mit einem Methanol-Benetzbarkeitsverfahren, wobei das Verfahren die folgenden Schritte umfasst:
- Hinzufügen und Einrühren von 0,2 g hydrophobem Siliciumdioxid in 50 ml wässrige Methanollösung verschiedener Konzentrationen;
- Bestimmen der Lösung mit der niedrigsten Methanolkonzentration unter den Methanollösungen, in denen sich die gesamte Menge des hydrophoben Siliciumdioxids abgesetzt hat;
- Verwenden der Methanolvolumenkonzentration der bestimmten Lösung als Hydrophobie des hydrophoben Siliciumdioxids.

2. Blutseparationszusammensetzung gemäß Anspruch 1, wobei das spezifische Gewicht bei 25°C 1,038 oder mehr und 1,095 oder weniger beträgt.

3. Blutseparationszusammensetzung gemäß Anspruch 1 oder 2, wobei das spezifische Gewicht bei 25°C 1,060 oder mehr und 1,090 oder weniger beträgt.

4. Blutseparationszusammensetzung gemäß einem der Ansprüche 1 bis 3, wobei die organische Komponente, die bei 25°C fließfähig ist, ein Harz enthält.

5. Blutseparationszusammensetzung gemäß Anspruch 4, wobei das Harz ein (Meth)acrylharz ist.

6. Blutseparationszusammensetzung gemäß einem der Ansprüche 1 bis 5, wobei das feine Siliciumdioxidpulver hydrophiles Siliciumdioxid enthält.

7. Blutseparationszusammensetzung gemäß einem der Ansprüche 1 bis 6, umfassend Silikonöl.

8. Blutprobenbehälter, umfassend:
einen Blutprobenbehälterkörper; und
die Blutseparationszusammensetzung gemäß einem der Ansprüche 1 bis 7,
wobei die Blutseparationszusammensetzung in dem Blutprobenbehälterkörper untergebracht ist.

9. Blutprobenbehälter gemäß Anspruch 8, umfassend ein wasserlösliches Dichtegradientenmaterial,
wobei das wasserlösliche Dichtegradientenmaterial in dem Blutprobenbehälterkörper untergebracht ist und
das wasserlösliche Dichtegradientenmaterial in dem Blutprobenbehälterkörper auf der Unterseite des Blutprobenbehälterkörpers in Bezug auf die Blutseparationszusammensetzung untergebracht ist.

10. Blutprobenbehälter gemäß Anspruch 9, wobei es sich bei dem wasserlöslichen Dichtegradientenmaterial um Ficoll handelt.

11. Blutprobenbehälter gemäß Anspruch 9 oder 10, wobei die Dichte des wasserlöslichen Dichtegradientenmaterials bei 20°C 1,083 g/ml oder mehr und 1,085 g/ml oder weniger beträgt.

12. Verfahren zum Trennen von Leukozyten unter Verwendung des Blutprobenbehälters gemäß einem der Ansprüche 8 bis 11,
wobei das Verfahren einen Zentrifugationsschritt zum Zentrifugieren des Blutprobenbehälters, aus dem Blut entnommen wird, umfasst.

## Revendications

1. Composition de séparation du sang comportant :
un composant organique ayant une fluidité à 25°C et de la silice en poudre fine,
**caractérisée en ce que** la silice en poudre fine inclut de la silice hydrophobe présentant une hydrophobicité de 60 % ou plus lorsqu'elle est mesurée par un procédé de mouillabilité au méthanol comportant les étapes suivantes consistant à :
- ajouter et agiter 0,2 g de silice hydrophobe dans 50 ml de solutions de méthanol aqueux de diverses concentrations ;
- déterminer la solution ayant la plus faible concentration de méthanol dans laquelle la totalité de la quantité de la silice hydrophobe s'est déposée ;
- prendre la concentration volumique de méthanol de la solution déterminée comme l'hydrophobicité de la silice hydrophobe.

2. Composition de séparation du sang selon la revendication 1, dans laquelle la densité à 25°C est de 1,038 ou plus et de 1,095 ou moins.

3. Composition de séparation du sang selon la revendication 1 ou 2, dans laquelle la densité à 25°C est de 1,060 ou plus et de 1,090 ou moins.

4. Composition de séparation du sang selon l'une quelconque des revendications 1 à 3, dans laquelle le composant organique ayant une fluidité à 25 °C contient une résine.

5. Composition de séparation du sang selon la revendication 4, dans laquelle la résine est une résine (méth)acrylique.

6. Composition de séparation du sang selon l'une quelconque des revendications 1 à 5, dans laquelle la silice en poudre fine inclut de la silice hydrophile.

7. Composition de séparation du sang selon l'une quelconque des revendications 1 à 6, comportant de l'huile de silicone.

8. Récipient de prélèvement sanguin comportant :
un corps de récipient de prélèvement sanguin ; et
la composition de séparation du sang selon l'une quelconque des revendications 1 à 7,
la composition de séparation du sang étant reçue dans le corps de récipient de prélèvement sanguin.

9. Récipient de prélèvement sanguin selon la revendication 8, comportant une matière à gradient de densité soluble dans l'eau,
dans lequel la matière à gradient de densité soluble dans l'eau est reçue dans le corps de récipient de prélèvement sanguin, et
la matière à gradient de densité soluble dans l'eau est reçue dans le corps de récipient de prélèvement sanguin sur un côté de fond du corps de récipient de prélèvement sanguin par rapport à la composition de séparation du sang.

10. Récipient de prélèvement sanguin selon la revendication 9, dans lequel la matière à gradient de densité soluble dans l'eau est le Ficoll.

11. Récipient de prélèvement sanguin selon la revendication 9 ou 10, dans lequel une densité à 20 °C de la matière à gradient de densité soluble dans l'eau est de 1,083 g/ml ou plus et de 1,085 g/ml ou moins.

12. Procédé pour séparer des leucocytes en utilisant le récipient de prélèvement sanguin selon l'une quelconque des revendications 8 à 11,
le procédé comportant une étape de centrifugation consistant à centrifuger le récipient de prélèvement sanguin à partir duquel du sang est prélevé.
